# EUROPÄISCHE PATENTSCHRIFT

(11) **EP 3 655 079 B1**
(45) Veröffentlichungstag und Bekanntmachung des Hinweises auf die Patenterteilung: **16.06.2021**
(21) Anmeldenummer: 18742702.6
(22) Anmeldetag: 22.06.2018
(51) Int. Cl.: A61M 15/08, A61M 15/00

(54) **EINZELDOSIS-PULVERINHALATOR**
SINGLE-DOSE POWDER-MEDICATION INHALER
INHALATEUR DE POUDRE À DOSE UNITAIRE

(30) Priorität: 18.07.2017 DE 102017006763
(43) Veröffentlichungstag der Anmeldung: 27.05.2020
(73) Patentinhaber: Perlen Packaging AG, Perlen, 6035 Perlen (CH)
(72) Erfinder: BELLER, Klaus-Dieter, 79341 Kenzingen (DE); KAISER, Bernd, 79346 Endingen (DE)
(74) Vertreter: mepat Patentanwälte
(86) Internationale Anmeldenummer: PCT/EP2018/000313
(87) Internationale Veröffentlichungsnummer: WO 2019/015796

(56) Entgegenhaltungen:
- EP-A1- 1 488 819
- WO-A1-89/07464
- WO-A2-2013/036881
- DE-A1-102014 005 646
- US-A- 4 841 964
- US-A1- 2002 108 611
- US-A1- 2012 132 204

## Beschreibung

Die Erfindung betrifft einen Einzeldosis-Pulverinhalator nach dem Oberbegriff des Anspruchs 1.

Die am Markt üblichen Pulverinhalatoren ("Inhalator") sind häufig zur Mehrfachanwendung konzipiert und daher aufwändig und teuer in der Herstellung. Die Anwendung als Einweg-Artikel ist oft nicht geeignet. Die Mehrfachanwendung schafft aber zum Teil große hygienische Probleme, da der Patient den Inhalator nach dem Gebrauch nicht korrekt, selten oder gar nicht reinigt. Problematisch ist auch, dass Abdeck-Kappen verloren gehen. Es ist aus hygienischer Sicht auch ungünstig, wenn der Patient den Inhalator falsch benutzt, etwa in den Inhalator ausatmet: Bei bekannten Inhalatoren atmen Patienten nicht immer in vorgegebener Expirationsrichtung, sondern durch den Inhalator aus, so dass der Inhalator infolge der Atemluftfeuchtigkeit bei der Mehrfachanwendung zu Verstopfung neigt, da das pulverförmige Arzneimittel verklumpt und im Inhalator im Luftkanal kleben bleibt. Diese Ablagerungen und Verklebungen, die auch mit pathologischen Keimen belastet sein können, können zu Dosierungenauigkeiten und weiteren Nachteilen führen, insbesondere bei der nasalen Applikation: Dort sind Nasenkeime zu berücksichtigen, z. B. wenn sich eine Nasennebenhöhlenentzündung als therapieresistent erweist. Möglicherweise steckt sich der Betroffene über den Inhalator (nasal, oropharyngeal) immer wieder selbst an.

Die WO 2007/042822 beschreibt einen einfach aufgebauten Einzeldosis-Pulverinhalator, der ein erstes Gehäuseteil und ein zweites Gehäuseteil aus Metall aufweist, die zusammengefügt ein Inhalatorgehäuse mit Auslass bilden. Das erste Gehäuseteil umfasst eine Medikamentenkammer, die eine Einzeldosis eines pulverförmigen Medikaments enthält und die durch eine an dem ersten Gehäuseteil befestigte Folie verschlossen ist. Diese Folie erstreckt sich aus dem Inhalatorgehäuse, so dass sie zur Verwendung durch einen Anwender gegriffen und abgezogen werden kann, wodurch das pulverförmige Medikament aus der Medikamentenkammer freigesetzt wird. Das zweite Gehäuseteil weist einen Sammelschacht und/oder eine Dispersionskammer auf, durch die ein in dem Inhalatorgehäuse ausgebildeter Luftkanal zu dem Auslass führt. Nach Öffnung der Medikamentenkammer wird bei Inhalation am Auslass durch den Spalt zwischen den Gehäuseteilen, der nach dem Abziehen der Folie verbleibt, ein Luftstrom erzeugt, der das aus der Medikamentenkammer freigesetzte pulverförmige Medikament aus dem Sammelschacht bzw. der Dispersionskammer mitreißt. Allerdings ist dieser Inhalator nicht zur nasalen Applikation vorgesehen und ausgebildet.

Aus DE 10 2014 017 409 A1 ist ein ebenfalls einfach aufgebauter Einzeldosis-Pulverinhalator bekannt, der aus einem Inhalatorgehäuse besteht, das ein Gehäuseteil aufweist, in dem zumindest eine Medikamentenkammer zur Aufnahme eines pulverförmigen Medikaments ausgeformt ist. Das Inhalatorgehäuse weist eine Auslassöffnung und einen Auslasskanal auf, der sich von der Medikamentenkammer zu der Auslassöffnung erstreckt. Eine Lufteinlassöffnung auf einer von der Auslassöffnung abgewandten Seite der Medikamentenkammer und ein Einlasskanal, der sich von der Lufteinlassöffnung zu der Medikamentenkammer erstreckt, sind ebenfalls in dem Gehäuseteil ausgeformt, das nach Befüllung der Medikamentenkammer mit einer Pulverdosis mit einem Deckelungselement gedeckelt wird. Weiter wird dort eine Ausführungsform gezeigt, in der zur nasalen Applikation die Auslassöffnung des Inhalators mit einem sich verjüngenden Nasenrüssel verbunden ist. Allerdings ist der Inhalator an sich in erster Linie für die orale Applikation vorgesehen und dementsprechend vor allem auch hinsichtlich der Desagglomeration des enthaltenen Pulvers ausgelegt.

GB 2 460 281 A offenbart einen Inhalator aus zumindest drei aufeinander laminierten Schichten mit einer Dicke von weniger als 3 mm. Die mittlere Schicht weist Ausschnitte auf, um zusammen mit einer Deck- und einer Bodenschicht einen Luftkanal, zumindest eine Medikamentenkammer und einen Luftauslassbereich zu begrenzen. Die Deckschicht kann eine Lufteinlassöffnung, die über einem Einlassbereich des Luftkanals der mittleren Schicht zu liegen kommt, und Entlüftungsöffnungen haben, die über jeder Medikamentenkammer zu liegen kommen. Lufteinlässe können auch zwischen Boden und Deckschicht durch Ausnehmungen in der mittleren Schicht auf der von dem Auslass abgewandten oder benachbarten Seite vorliegen, von wo gerade oder gewinkelte Luftkanäle in die Medikamentenkammer münden. Ferner kann ein Auslassabschnitt des Luftkanals zwischen der Medikamentenkammer und der Auslassöffnung gewunden sein DE 10 2014 005646 offenbart ebenfalls einen Einzeldosis-Pulverinhalator zur nasalen Applikation.

Ein Pulverinhalator, der aus drei Gehäuseteilen besteht, ist aus WO 2013/036881 A2 bekannt. Zwei Gehäuseteile umschließen Lufteinlassbereich und die Medikamentenkammer; der Pulver-Luftauslassbereich zum Mundstück hin ist ein separates Bauteil. Dort ist in einem Gehäuseteil eine Ringkammer für das Pulver ausgebildet, in dem anderen sind Lufteinlässe, die nach Zusammenfügen in die Ringkammer münden, und ein Auslassgitter ausgebildet. Über dem Auslassgitter wird das separate Auslass-Mundstück befestigt. Die Lufteinlässe sind so ausgerichtet, dass Luft auf den Boden der Ringkammer unter einem nicht-tangentialen Winkel gerichtet wird, wobei insbesondere zwei Lufteinlässe vorgeschlagen sind, die aus entgegengesetzten Richtungen in die Ringkammer münden. Um zu verhindern, dass beim Neigen des Inhalators Pulver aus einer Lufteinlassöffnung verloren geht, kann der Lufteinlasskanal eine zusätzliche Windung haben.

Therapeutisch wird nasaler und oraler Applikationsweg vor allem hinsichtlich der Depositionsziele für das pulverförmige Medikament unterschieden. So zielt die nasale Applikation auf die Schleimhäute des Nasopharynx, die oral inhalative Anwendung auf die Schleimhäute des Mund-Rachen-Raums und beide auf die Deposition der Aerosolpartikel in der Lunge nach der Inhalation über die Nase oder den Mund. Hierbei kann außer einer lokal begrenzten auch eine systemische therapeutische Wirkung erzielt werden. Wo die Deposition der inhalierten Pulverpartikel stattfindet, hängt unter anderem von der Partikelgröße ab.

Ausgehend von diesem Stand der Technik ist es Aufgabe der vorliegenden Erfindung, einen besonders kompakt gestalteten Einzeldosis-Pulverinhalator zu schaffen, der insbesondere für die nasale Anwendung optimiert ist.

Diese Aufgabe wird durch einen Einzeldosis-Pulverinhalator mit den Merkmalen des Anspruchs 1 gelöst. Weiterbildungen sind in den Unteransprüchen ausgeführt.

Der erfindungsgemäße Einzeldosis-Pulverinhalator ist besonders vorteilhaft auch für die nasale Applikation von pulverförmigen Medikamenten vorgesehen. Bei der nasalen Applikation ist eine Deposition vor allem auf den Schleimhäuten des Nasopharynx vorgesehen. Partikelgrößen im Bereich von 3 bis 6 µm sind dabei geeignet; Abweichungen nach oben und unten sind zulässig

Der erfindungsgemäße Einzeldosis-Pulverinhalator hat daher ein flaches Inhalatorgehäuse, das aus nur zwei flächigen Gehäuseteilen besteht, die entlang einer Verbindungsebene miteinander verbunden sind. In den Gehäuseteilen ist ein durch eine beidseitige Wandung begrenzter Luftkanal ausgeformt, der sich um eine ausgeformte Pulveraufnahmemulde für eine pulverfömige Medikamentendosis zu einer Medikamentenkammer aufweitet. Der Luftkanal erstreckt sich dabei zwischen einer Lufteinlassöffnung und einer Auslassöffnung, die in den Gehäuseteilen ausgebildet bzw. ausgeformt sind, wobei die Lufteinlassöffnung nicht auf einer von der Auslassöffnung abgewandten Seite des Inhalators angeordnet ist, d. h. dass gedachte Achsen durch Lufteinlass- und Auslassöffnung weder fluchten noch parallel sind. Der Verlauf des Luftkanals stellt dabei eine Richtungsänderung einer Luftströmung durch die Lufteinlassöffnung in Bezug zu der Auslassöffnung bereit und weist einen Auslassabschnitt zwischen der Medikamentenkammer und der Auslassöffnung auf, der mehr als eine Windung aufweist. D. h., dass bei einem erfindungsgemäßen Einzeldosis-Pulverinhalator die Lufteinlassöffnung und die Auslassöffnung derart in Bezug zueinander angeordnet sind, dass eine Lufteinlassströmungsrichtung, die durch die Anordnung der Lufteinlassöffnung definiert wird, von einer Luftauslassströmungsrichtung abweicht, die durch die Anordnung der Auslassöffnung definiert wird, sodass die ausströmende Luft den Inhalator nicht in Geradeausrichtung in Bezug auf die einströmende Luft verlässt, d. h., dass Lufteinlassöffnung und Auslassöffnung eben nicht an voneinander abgewandten Seiten des Inhalators angeordnet sind. Diese von der "Geradeausrichtung" abweichende Anordnung von Lufteinlassöffnung und Auslassöffnung ist bei einer nasalen Anwendung wichtig, da hier der Inhalator anders als bei der oralen Anwendung so zu halten ist, dass die Auslassöffnung im Wesentlichen senkrecht nach oben - in die nach Nasenlöcher - weist, sodass durch die abweichende Anordnung der Lufteinlassöffnung vermieden wird, dass diese bei der Anwendung nach unten weist. Vorteilhaft kann dadurch bei der Anwendung kein Pulver durch die Lufteinlassöffnung verloren gehen, was bei einem herkömmlichen Pulverinhalator, bei dem die Lufteinlassöffnung auf einer von der Auslassöffnung abgewandten Seite angeordnet ist, passieren könnte, wenn bei der nasalen Anwendung mit der Auslassöffnung an den Nasenlöchern die Lufteinlassöffnung senkrecht nach unten weist.

Dabei weist der erfindungsgemäße Einzeldosis-Pulverinhalator zur nasalen Applikation ein an die Auslassöffnung angeformtes Nasenstück auf, das zur Aufnahme in ein oder beide Nasenlöcher ausgebildet ist. Das Nasenstücks zur Aufnahme in nur ein Nasenloch zu gestalten macht insbesondere in Kombination mit zwei Pulveraufnahmemulden für zwei Teildosen Sinn. Zur Steuerung der Strömungsgeschwindigkeit kann sich ein an die Lufteinlassöffnung anschließender Abschnitt des Einlasskanals von der Lufteinlassöffnung in Richtung Medikamentenkammer verjüngen. Der in die Auslassöffnung mündende Abschnitt des Auslasskanalsverjüngt sich in Richtung der Auslassöffnung auf den Querschnitt des Nasenstücks.

Es ist nicht ausgeschlossen, dass ein erfindungsgemäßer Einzeldosis-Pulverinhalator zur oral-inhalativen Anwendung verwendet werden kann - wenn etwa eine Deposition des Pulvers in der Mund- oder Rachenschleimhaut gewünscht ist. Gegebenenfalls kann hierfür zusätzlich ein Mundstück vorgesehen sein, das auf das Nasenstück aufgesetzt werden kann.

In der Anwendung des Inhalators bewirkt der Anwender durch einen Einatmungszug an der Auslassöffnung einen Luftstrom durch den Inhalator, sodass die Pulverpartikel, die in der Pulveraufnahmemulde in einer vorbestimmten Medikamentendosis bereitgestellt sind, mit dem Luftstrom mitgerissen werden. Damit die Pulverpartikel auf dem Weg zwischen der Medikamentenkammer und der Auslassöffnung gleichmäßig und gut im Luftstrom verteilt werden, ehe sie vom Anwender inhaliert werden, weist ein Auslassabschnitt des Luftkanals, der zwischen der Medikamentenkammer und der Auslassöffnung liegt, mehr als eine Windung auf, sodass die in der Anwendung durch den Auslasskanalabschnitt geführte Luftströmung mehrere Richtungsänderungen vollführt. Auf zusätzliche Desagglomerationsstrukturen wird in dem erfindungsgemäßen Einzeldosis-Pulverinhalator verzichtet, da eine weitere Zerkleinerung der im Luftstrom mitgerissenen und dabei verwirbelten Pulverpartikel nicht erforderlich und auch nicht wünschenswert ist.

Vorteilhaft besteht der erfindungsgemäße Pulverinhalator lediglich aus zwei Gehäuseteilen, in denen alle relevanten Abschnitte ausgeformt sind. Der Pulverinhalator lässt sich daher äußerst kostengünstig fertigen und ist somit zur Verabreichung einer Pulverdosis in einer Einmalanwendung mit den damit verbundenen hygienischen Vorteilen für den Anwender geeignet.

Die Anordnung der Lufteinlassöffnung und der Auslassöffnung ist dabei in Bezug zueinander derart gestaltet, dass zwischen der durch die Anordnung der Lufteinlassöffnung definierten Lufteinlassströmungsrichtung und der durch die Anordnung der Auslassöffnung definierten Luftauslassströmungsrichtung ein Winkel in einem Bereich von 90° (senkrecht zur Geradeausrichtung) und 180° (quasi eine Kehrtwendung) gebildet wird. Mit einem Winkel zwischen den Strömungsrichtungen von 180° ist somit gemeint, dass die Luftströmungsrichtungen am Einlass und am Auslass entgegengesetzt sind, folglich an der gleichen Seite des Pulverinhalators vorliegen. So sind die Lufteinlassöffnung und die Auslassöffnung entweder nebeneinander an der gleichen Seite oder um 90° an benachbarten Seiten oder um einen dazwischen liegenden Winkel versetzt zueinander angeordnet - nicht jedoch an abgewandten Seiten. Zwar sind auch kleinere Winkel als 90° zum Abweichen von der Geradeausrichtung denkbar, allerdings ist zur effektiven Verhinderung des ungewollten Pulveraustritts aus der Lufteinlassöffnung eine Abweichung von der Geradeausrichtung von zumindest 90° sinnvoll, um eine im Gebrauch nach unten weisende Lufteinlassöffnung zu vermeiden.

Die Anordnung der Lufteinlassöffnung und der Auslassöffnung am erfindungsgemäßen Einzeldosis-Pulverinhalator kann auch dahingehend variieren, dass die Lufteinlassöffnung und die Auslassöffnung beide senkrecht oder beide parallel zu der Verbindungsebene der Gehäuseteile angeordnet sind. Auch kann vorgesehen sein, dass eine der beiden Öffnungen, Lufteinlassöffnung und Auslassöffnung, in einer zur Verbindungsebene parallelen Ebene und die andere Öffnung senkrecht dazu angeordnet ist.

Besonders bevorzugt sind die Varianten, bei denen Lufteinlass- und -auslassöffnung nebeneinander angeordnet sind, weil es dann nahezu keinen Bedienfehler geben kann - Pulver kann im Grunde nicht herausfallen, egal, wie der Inhalator während der Anwendung gehalten wird.

Bezüglich der Gestaltung des Auslassabschnitts des Luftkanals zwischen der Medikamentenkammer und der Auslassöffnung eines erfindungsgemäßen Einzeldosis-Pulverinhalators kann ein mäandrierender Kanal mit zumindest drei Kurven als Windungen vorgesehen sein, wobei 90°- oder 180°-Kurven oder eine Kombination aus beiden bevorzugt sein kann. Im mäandrierenden Kanal erfährt der Luftstrom dabei mehrfache Richtungswechsel. Alternativ zu einem mäandrierenden Kanal kann der Auslassabschnitt auch als spiralförmiger Kanal mit zumindest zwei Windungen ausgebildet sein. In dem spiralförmigen Kanal erfährt der Luftstrom eine kontinuierliche Richtungsänderung. Anzahl und Krümmungsgrad der Windungen im mäandrierenden und spiralförmigen Kanal hängen auch von der Anordnung der Auslassöffnung in Bezug auf die Lage der Medikamentenkammer ab. Ein spiralförmiger Kanal hat eine ideale Zyklonwirkung, also eine besonders gute Luftverwirbelungseigenschaft.

Damit das pulverförmige Medikament bis zur Anwendung in der Pulveraufnahmemulde bleibt und dort während der Aufbewahrung oder beim Mitführen des Einzeldosis-Pulverinhalators vor Umwelteinflüssen geschützt ist, wird in einer bevorzugten Ausführungsform die Pulveraufnahmemulde nach Befüllung mit der Pulverdosis verschlossen. Hierzu ist ein Verschlusselement vorgesehen, welches einfach durch den Anwender vor der Anwendung entfernt werden kann. Als lösbarer Verschluss für die Pulveraufnahmemulde kann der Einzeldosis-Pulverinhalator ein abziehbares Folienelement aufweisen. Eine Verschlusslasche des Folienelements verschließt die eine Pulveraufnahmemulde in der Medikamentenkammer und von der Verschlusslasche erstreckt sich aus der Medikamentenkammer und aus dem Inhalator heraus eine Abziehlasche des Folienelements, die der Anwender greifen kann, so dass er durch Zug an der Abziehlasche durch die Verschlusslasche von der Pulveraufnahmemulde abziehen und so den Inhalator anwendungsbereit machen kann.

In einer Ausführungsform kann vorgesehen sein, dass sich die Abziehlasche des Folienelements durch einen Einlassabschnitt des Luftkanals zwischen der Lufteinlassöffnung und der Medikamentenkammer aus der Lufteinlassöffnung erstreckt. Der Einlassabschnitt verläuft dabei in gerader Linie zwischen Medikamentenkammer und der Einlassöffnung.

In einer dazu alternativen Ausführungsform kann der Inhalator einen zusätzlichen Laschenkanal aufweisen, der sich in gerader Linie von der Medikamentenkammer bis zu einer Laschenöffnung erstreckt, so dass die Abziehlasche des Folienelements sich hier durch den Laschenkanal und aus der Laschenöffnung hinaus erstrecken kann. Die Laschenöffnung ist wie die Lufteinlassöffnung derart in Bezug zur Auslassöffnung angeordnet, dass aus der Laschenöffnung in der Anwendung des Inhalators mit der Auslassöffnung an der Nase des Anwenders nach Entfernung des Folienelements kein Pulver aus der Medikamentenkammer durch den Laschenkanal und aus der Laschenöffnung rieseln kann. Tatsächlich wirkt die Laschenöffnung nach Entfernung des Folienelements auch als zusätzliche Lufteinlassöffnung, wodurch in der Anwendung zwei Luftströmungen in die Medikamentenkammer eintreten und damit das Aufwirbeln und Mitreißen der Pulverpartikel aus der Pulveraufnahmemulde noch verbessert werden kann.

Um sicherzustellen, dass auch nach Entfernung des Verschlusselements beim Hantieren mit einem erfindungsgemäßen Einzeldosis-Pulverinhalator kein Pulver aus der Lufteinlassöffnung bzw. der Laschenöffnung, sofern vorhanden, herausfallen kann, kann der Einlassabschnitt des Luftkanals bzw. der Laschenkanal eine Pulverrückhaltestruktur aufweisen, die den Kanalquerschnitt verengt. Vorzugsweise kann eine solche Pulverrückhaltestruktur durch einen Wall gebildet werden, der sich quer zur Strömungsrichtung in den Luftkanal (bzw. Laschenkanal) erstreckt. Besonders bevorzugt kann die Pulverrückhaltestruktur durch mehrere, gegenüber- und/oder nebeneinanderliegend alternierend angeordnete Wälle bzw. Wallabschnitte gebildet werden, so dass der Weg durch die Pulverrückhaltestruktur schikanen- oder labyrinthartig verläuft.

Ferner kann auch der Einlassabschnitt des Luftkanals - wie der Auslassabschnitt - mehr als eine Windung aufweisen, sodass auch die durch den Einlasskanalabschnitt führbare Luftströmung mehrere Richtungsänderungen vollführt, sodass eine turbulente Luftströmung in die Medikamentenkammer eintritt, um das dort in der Pulveraufnahmemulde vorliegende Pulver aufzuwirbeln und mitzureißen. Gerade in dieser Ausführungsform des Pulverinhalators mit einem gewundenen Einlassabschnitt ist es sinnvoll, einen Laschenkanal zum Abziehen des Folienelements vorzusehen, da ein gewundener Einlasskanal das Durchziehen des Folienelements nicht oder nur bedingt zulässt.

Der erfindungsgemäße Einzeldosis-Pulverinhalator ist zum Einmalgebrauch vorgesehen. Dies heißt allerdings nicht, dass die Pulverdosis nur in einer einzigen Pulveraufnahmemulde vorgelegt sein muss. Vielmehr kann insbesondere bei der nasalen Anwendung für eine therapeutische Verabreichung eine Applikation zweier Teildosen einzeln in jedes der Nasenlöcher vorgesehen sein. So können in einem erfindungsgemäßen Pulverinhalator in den Gehäuseteilen auch zwei Pulveraufnahmemulden für jeweils eine pulverfömige Medikamenten(teil-)dosis in der Medikamentenkammer ausgebildet sein.

Auch hier kann jede Pulveraufnahmemulde durch die Verschlusslasche jeweils eines abziehbaren Folienelements verschlossen sein, so dass in der Anwendung nacheinander pro Nasenloch eine Pulveraufnahmemulde geöffnet und das Pulver freigesetzt werden kann. Außerdem können zwei (oder auch mehr) Pulveraufnahmemulden in einem erfindungsgemäßen Inhalator für den Fall vorgesehen werden, in dem verschiedene Komponenten eines pulverförmigen Medikaments erst im Anwendungsfall vermischt werden dürfen. In diesem Fall können die unterschiedlichen Komponenten der Medikamentendosis in entsprechenden Pulveraufnahmemulden gelagert werden, die hier dann auch durch ein gemeinsames Folienelement verschlossen sein können, wenn die Komponenten gemeinsam inhaliert werden sollen.

Die beiden Gehäuseteile eines erfindungsgemäßen Einzeldosis-Pulverinhalators können aus Plattenelementen gebildet sein, in denen sämtliche Strukturen ausgeformt sind, sodass diese sich aus der ebenen Plattenbasis erheben. Die Gehäuseteile aus Plattenelementen können (nach Befüllung der Pulveraufnahmemulde) dann flächig in der Verbindungsebene außerhalb der ausgeformten Bereiche miteinander verbunden sein. Vorzugsweise, weil dadurch ein stabileres Gehäuse und eine bessere Handhabbarkeit für den Nutzer erreicht werden kann, können die beiden Gehäuseteile jedoch aus Schalenelementen mit umlaufenden Rändern gebildet sein, wobei die Gehäuseteile dann in der Verbindungsebene zumindest an diesen Rändern, vorzugsweise aber auch an den Kanal begrenzenden Wandungen miteinander verbunden sind.

Das Verbinden der Gehäuseteile nach dem Verschließen der Pulveraufnahmemulde mit einem Folienelement, wobei die Verschlusslasche an der Medikamentenkammer versiegelt oder verklebt wird und der zu der Abziehlasche führende Folienabschnitt gefaltet bzw. umgeklappt wird, kann durch Siegelung, Klebung oder auch durch Schweißen (z. B. Ultraschallschweißen) erfolgen.

Vorteilhaft kostengünstig können die Gehäuseteile des Pulverinhalators jeweils Kunststoffspritzgussteile oder aus einer pharmagerechten, bevorzugt sortenreinen Kunststofffolie geformt, bevorzugt tiefgezogen sein. Der verwendete Kunststoff zumindest eines der Gehäuseteile kann ein transparenter Kunststoff sein, so dass das in der Medikamentenkammer vorliegende Pulver und dessen Verabreichung durch Ansicht kontrolliert werden kann. Vorzugsweise werden für beide Gehäuseteile derselbe Kunststoff verwendet, sodass der Pulverinhalator nach Gebrauch gut recycelt werden kann.

Ferner kann vorgesehen sein, dass der zur Fertigung der Gehäuseteile eingesetzte Kunststoff biologisch abbaubar ist, falls er nicht dem Recycling zugeführt wird. Zu den biologisch abbaubaren Kunststoffen gehören verschiedene Polyester, Polyesteramide, Polyurethane und Polyvinylalkohol. Auch Polymilchsäure lässt sich biologisch abbaubar durch eine entsprechende Mischung der Ausgangsstoffe, die unterschiedlichen Isomere der Milchsäure, und gegebenenfalls als Copolymerisat einstellen.

Zum Schutz vor Plagiaten kann der Kunststoff ferner einen Marker enthalten, der sich am fertigen Pulverinhalator nachweisen lässt. Ferner kann, um erhöhten hygienischen Anforderungen zu entsprechen, der verwendete Kunststoff ein antiseptischer und/oder antimikrobieller Kunststoff sein. Alternativ hierzu kann zumindest entlang dem Luftkanal eine antimikrobielle Beschichtung vorgesehen sein.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine perspektivische Ansicht einer Ausführungsform eines Einzeldosis-Pulverinhalators mit entgegengesetzten Luftströmungsrichtungen durch Luftein- und Auslassöffnung in einer Ebene senkrecht zur Verbindungsebene,
- **Fig. 2**: eine Vorderansicht des Einzeldosis-Pulverinhalators aus Fig. 1,
- **Fig. 3**: eine Rückansicht des Einzeldosis-Pulverinhalators aus Fig. 1,
- **Fig. 4**: eine Draufsicht auf den Einzeldosis-Pulverinhalator aus Fig. 1,
- **Fig. 5**: eine Seitenansicht des Einzeldosis-Pulverinhalators aus Fig. 1,
- **Fig. 6**: eine perspektivische Ansicht einer weiteren Ausführungsform eines Einzeldosis-Pulverinhalators mit um 90° versetzten Luftströmungsrichtungen durch Luftein- und Auslassöffnung in unterschiedlichen Ebenen,
- **Fig. 7**: eine Vorderansicht des Einzeldosis-Pulverinhalators aus Fig. 6,
- **Fig. 8**: eine Seitenansicht des Einzeldosis-Pulverinhalators aus Fig. 6,
- **Fig. 9**: eine Draufsicht auf den Einzeldosis-Pulverinhalator aus Fig. 6,
- **Fig. 10**: eine perspektivische Ansicht einer weiteren Ausführungsform eines Einzeldosis-Pulverinhalators mit entgegengesetzten Luftströmungsrichtungen durch Luftein- und Auslassöffnung in einer Ebene parallel zur Verbindungsebene,
- **Fig. 11**: eine Rückansicht des Einzeldosis-Pulverinhalators aus Fig. 10,
- **Fig. 12**: eine Seitenansicht des Einzeldosis-Pulverinhalators aus Fig. 10,
- **Fig. 13**: eine perspektivische Ansicht einer noch weiteren Ausführungsform eines Einzeldosis-Pulverinhalators mit entgegengesetzten Luftströmungsrichtungen durch Luftein- und Auslassöffnung in einer Ebene parallel zur Verbindungsebene,
- **Fig. 14**: eine Rückansicht des Einzeldosis-Pulverinhalators aus Fig. 13,
- **Fig. 15**: eine Seitenansicht des Einzeldosis-Pulverinhalators aus Fig. 13,
- **Fig. 16**: eine perspektivische Ansicht einer nicht erfindungsgemäßen Ausführungsform eines Einzeldosis-Pulverinhalators mit um 90° versetzten Luftströmungsrichtungen durch Luftein- und Auslassöffnung,
- **Fig. 17**: eine Innenansicht eines Gehäuseteils des Einzeldosis-Pulverinhalators aus Fig. 16,

- **Fig. 18**: eine perspektivische Ansicht einer weiteren Ausführungsform eines Einzeldosis-Pulverinhalators mit um 90° versetzten Luftströmungsrichtungen,
- **Fig. 19**: eine perspektivische Ansicht einer weiteren Ausführungsform eines Einzeldosis-Pulverinhalators mit um 90° versetzten Luftströmungsrichtungen mit vertauschter Luftein- und Auslassöffnung,
- **Fig. 20**: eine Detailseitenschnittansicht durch eine Pulverrückhaltestruktur.

Die erfindungsgemäße Vorrichtung bezieht sich auf einen einfach und kostengünstig herstellbaren Einzeldosis-Pulverinhalator, dessen Gehäuse aus lediglich zwei Gehäuseteilen besteht und der für die nasale Anwendung optimiert ist. Der erfindungsgemäße Einzeldosis-Pulverinhalator kann in der Art einer Sichtverpackung bzw. blisterartig ausgeführt sein, wobei eines der Gehäuseteile oder beide transparent sein können und so einen Einblick in den Pulverinhalator, insbesondere in die Medikamentenkammer gestatten. So kann der Inhalt bzw. die vollständige Entleerung des Inhalts nach der Anwendung kontrolliert werden.

Das Gehäuse eines erfindungsgemäßen Einzeldosis-Pulverinhalators 10, wie er anhand einiger beispielhafter Ausführungsformen in den Figuren dargestellt ist, ist flach und klein dimensioniert - der Inhalator ist so klein, dass man ihn sich in etwa mit der Dimension eines Stapels aus 5 Scheckkarten vorstellen kann. Er kann damit gut in einer Handtasche oder sogar einer Jackentasche mitgeführt werden. Das Gehäuse besteht aus zwei flächigen Gehäuseteilen 1, 2, die entlang einer Verbindungsebene E miteinander verbundenen sind. In den Gehäuseteilen 1, 2 ist eine Medikamentenkammer 11 mit zumindest einer Pulveraufnahmemulde 11' für eine pulverfömige Medikamentendosis ausgeformt. In den mit den Figuren gezeigten Beispielen ist eine einzige Pulveraufnahmemulde 11' in einem Gehäuseteil 1 ausgeformt. Anders als dargestellt können aber auch mehr als eine Pulveraufnahmemulde für Teildosen oder erst bei Verwendung zu mischenden Komponenten vorgesehen sein. Eine zweite oder weitere Pulveraufnahmemulde kann in demselben Gehäuseteil 1 oder in dem anderen Gehäuseteil 2 ausgeformt sein.

Weiter sind in den Gehäuseteilen 1, 2 eine Lufteinlassöffnung 14 und eine Auslassöffnung 12 sowie ein durch eine beidseitige Wandung 7 begrenzter Luftkanal ausgeformt, der die Lufteinlassöffnung 14 mit der Medikamentenkammer 11 und diese mit der Auslassöffnung 12 verbindet. Die Anordnung der Lufteinlassöffnung 14 und der Auslassöffnung 12 in Bezug zueinander ist dabei derart gewählt, dass eine Lufteinlassströmungsrichtung L_{E}, die durch die Anordnung der Lufteinlassöffnung 14 definiert wird, nicht der Luftauslassströmungsrichtung L_{A} entspricht, die durch die Anordnung der Auslassöffnung 12 definiert wird, sodass die Lufteinlassöffnung 14 im Anwendungsfall, wenn der Nutzer die Auslassöffnung 12 nach oben weisend an den Nasenlöchern anordnet, nicht nach unten weist, und so kein Pulver aus der Lufteinlassöffnung 14 herausfallen kann.

Zudem weist der erfindungsgemäße Einzeldosis-Pulverinhalator 10 einen Auslasskanalabschnitt 13 auf, der sich mehr als einer Windung 6 von der Medikamentenkammer 11 bis zur Auslassöffnung 12 erstreckt, sodass die durch den Auslasskanalabschnitt 13 geführte Luftströmung mehrere Richtungsänderungen unterzogen wird und damit eine turbulente Luftströmung erzielt wird, mit der das in der Medikamentenkammer 11 mitgerissene Pulver gleichmäßig verteilt mittransportiert wird. Für die nasale Anwendung sind zudem keine Desagglomerationsstrukturen im Auslasskanal erforderlich oder wünschenswert, wenn die Pulverpartikel in der Nasenschleimhaut abgeschieden werden sollen, und daher nicht weiter zerkleinert werden sollen.

Die Ausführungsbeispiele betreffen verschiedene Varianten bezüglich der Anordnung von Lufteinlassöffnung 14 und Auslassöffnung 12 sowie die Gestaltung des gewundenen Auslasskanalabschnitts 13.

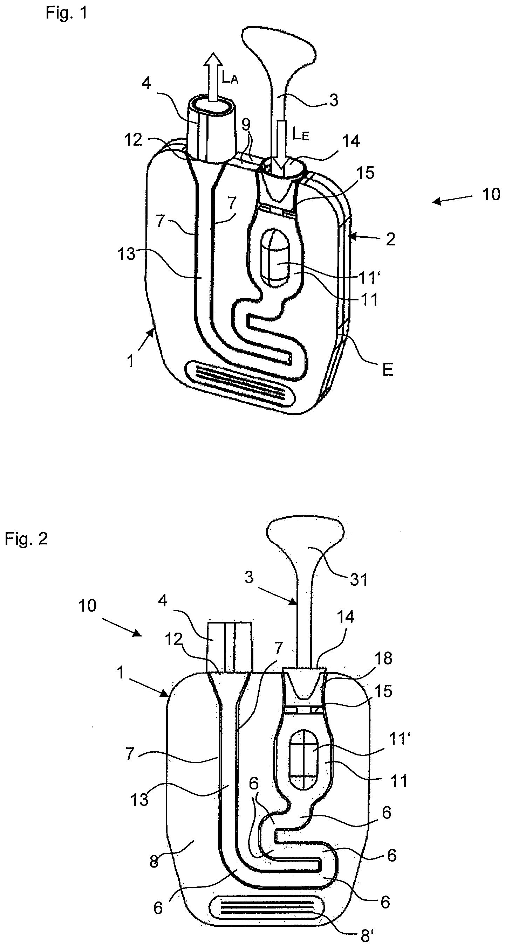

**Fig. 1 bis 5** zeigen eine erste beispielhafte Ausführungsform eines erfindungsgemäßen Einzeldosis-Pulverinhalators 10, bei dem die Lufteinlassöffnung 14 und die Auslassöffnung 12 auf der gleichen Seite am Rand 9 der Gehäuseteile 1, 2 angeordnet sind, in verschiedenen Ansichten.

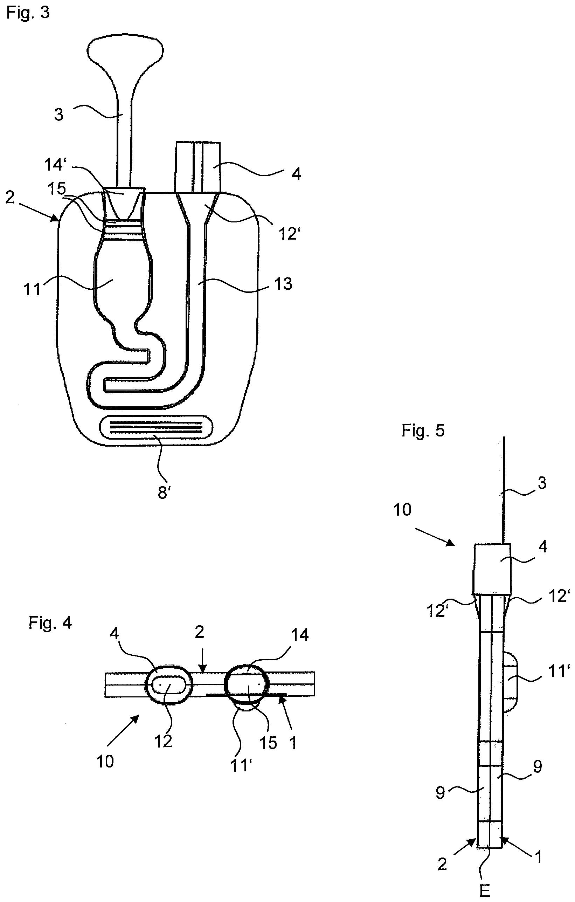

In **Fig. 1 und 2** ist das Gehäuseteil 1 gut zu sehen, das die in der Medikamentenkammer 11 ausgeformte Pulveraufnahmemulde 11' aufweist. Die Gehäuseteile 1, 2, die eine Schalenbasis 8 aufweisen, sind über ihre Ränder 9 in der Verbindungsebene E bis auf die Lufteinlassöffnung 14 und die Auslassöffnung 12 verbunden, wie dies auch in **Fig. 4 und 5** zu sehen ist. Zwar ist die Auslassöffnung 12 nur in **Fig. 4** sichtbar, jedoch auch in den anderen Figuren indiziert, um die Lage der Auslassöffnung 12 am Rand 9 der Gehäuseteile 1, 2 zu verdeutlichen. An die Auslassöffnung 12 ist ein Nasenstück 4 angeformt, das in den dargestellten Beispielen zur Anordnung an beide Nasenlöcher konzipiert ist. In **Fig. 4** sieht man, wie eine Auslassöffnung 12 für ein Nasenloch einen Luftstrom bereitstellt. Die Auslassöffnung 12 dort kann durch einen Rand aus zwei sich überlappenden Halbschalen gebildet werden, so dass ein elastischer Rand mit einer Federwirkung gebildet wird, der sich von innen an ein Nasenloch anlegen kann.

Grundsätzlich - aber figurativ nicht gezeigt - kann das Nasenstück auch doppelläufig ausgebildet sein, um durch beide Nasenlöcher genutzt zu werden. Dabei kann sich der Luftstrom aus dem Auslasskanalabschnitt 13, der zur Auslassöffnung 12 hin eine Aufweitung 12' zur Strömungsverlangsamung aufweist, in zwei Teilströme aufteilen.

Nicht in den Figuren dargestellt sind Varianten eines Nasenstücks, die zur Aufnahme in ein Nasenloch oder mit zwei Rüsselfortsätzen für beide Nasenlöcher ausgebildet sind, die aber dennoch Nasenstücke im Sinne der Erfindung darstellen.

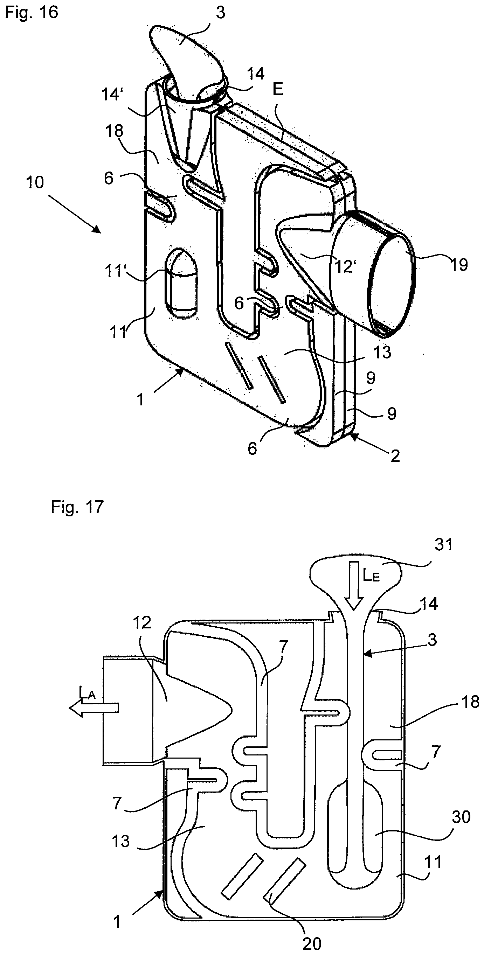

Wie in **Fig. 1** durch die Blockpfeile angedeutet, verlaufen die Luftströmungsrichtung L_{E} an der Lufteinlassöffnung 14 und die Luftströmungsrichtung L_{A} an der (durch das Nasenstück 4 ergänzten) Auslassöffnung 12 in entgegengesetzten Richtungen, sodass die Lufteinlassöffnung 14 im Anwendungsfall mit dem Nasenstück 4 an den Nasenlöchern eines Benutzers ebenfalls nach oben weist. Vor der Anwendung ist zunächst das Folienelement 3, das die Pulveraufnahmemulde 11' verschließt, zu entfernen. Das Folienelement 3 besteht aus einer Verschlusslasche 30, die die Pulveraufnahmemulde 11' verschließt, und einer Abziehlasche 31, die über einen Bandabschnitt mit der Verschlusslasche 30 verbunden ist (vgl. **Fig. 17****)** und durch die Lufteinlassöffnung 14 aus dem Inhalator herausragt. Zur Anwendung greift ein Benutzer die Abziehlasche 31 und zieht daran, um die Verschlusslasche 30 von der Pulveraufnahmemulde 11' abzuziehen und das Folienelement 3 aus dem Inhalator 10 zu entfernen.

Der Benutzer erzeugt dann durch einen Einatmungszug mit der Nase an dem Nasenstück 4 eine Luftströmung durch den im Inhalator 10 bereitgestellten Luftkanal, der aus einem Einlasskanalabschnitt 18, der Medikamentenkammer 11 und dem Auslasskanalabschnitt 13 gebildet wird, die durch Wandungen 7 begrenzt sind. In den Figuren sind auch diese innenliegenden Wandungen 7 des Luftkanals dargestellt, wenngleich diese von außen nicht sichtbar sein müssen, wenn etwa der Inhalator aus opakem Material gefertigt ist.

Die beim Einatemzug am Nasenstück durch die Lufteinlassöffnung 14 eintretende Luftströmung gelangt durch den Einlasskanalabschnitt 18 in die Medikamentenkammer 11 und reißt dort das nach Abziehen des Folienelements 3 freigelegte Pulver mit, durch den Auslasskanalabschnitt 13, der in die Auslassöffnung 12 mündet. Der Auslasskanalabschnitt 13 ist hier als mäandrierender Kanal mit mehreren Kurven 6 ausgebildet. Es versteht sich von selbst, dass sich ein mäandrierender Kanal im Sinne der Erfindung auch von dem abgebildeten Verlauf, hinsichtlich Anzahl, Krümmung, Radius und Richtung der Kurven, unterscheiden kann, ohne den Schutzumfang zu verlassen.

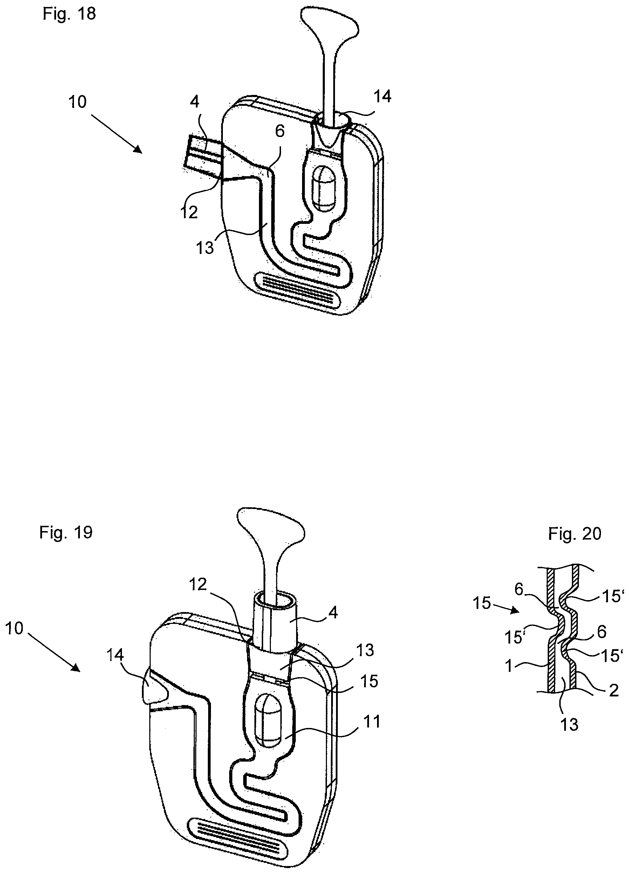

Die Einlassöffnung 14 weist nach außen eine trichterförmige Aufweitung 14' auf, vor allem in Bezug auf die Schalenbasis 8 der Gehäuseteile 1, 2, wie **Fig. 3 und 4** entnehmbar ist. Im sich an die Einlassöffnung 14 anschließenden Einlasskanalabschnitt 18 befindet sich eine Pulverrückhaltestruktur 15, die das Risiko des versehentlichen Austretens von Pulver bei der Handhabung des Inhalators 10 beim und nach dem Abziehen des Folienelements 3 und vor der Anordnung an der Nase deutlich verringert. Die Pulverrückhaltestruktur 15 besteht in den gezeigten Beispielen aus Wällen 15' (vgl. Schnittdarstellung **Fig. 20),** die sich alternierend von beiden Gehäuseteilen 1, 2 quer zur Strömungsrichtung in den Luftkanal erstrecken und damit ein schikanenartiges Hindernis darstellen und den Querschnitt des Einlasskanalabschnitts an dieser Stelle verengen, wie dies auch in **Fig. 4** zu sehen ist. Im gezeigten Beispiel weist Gehäuseteil 1 einen Wall 15' und Gehäuseteil 2 zwei versetzt dazu angeordnete Wälle 15' auf. Auch hier sind ohne weiteres Abweichungen einer Pulverrückhaltestruktur hinsichtlich Anzahl und Formverlauf der Wälle denkbar, wenn sie auch nicht explizit hier beschrieben sind.

Auf einer von der Auslassöffnung 12 / dem Nasenstück 4 abgewandten Seite des Pulverinhalators 10 ist in der Schalenbasis 8 der Gehäuseteile 1, 2 jeweils eine Riffelungstruktur 8' an der Oberfläche vorgesehen, die einer verbesserten Griffigkeit des Inhalators 10 beim Halten durch einen Benutzer dient.

Für die im Zusammenhang mit den weiteren Figuren beschriebenen Ausführungsformen werden im Folgenden nur die Merkmale beschrieben, die sich von dem mit **Fig. 1 bis 5** beschriebenen Beispiel unterscheiden.

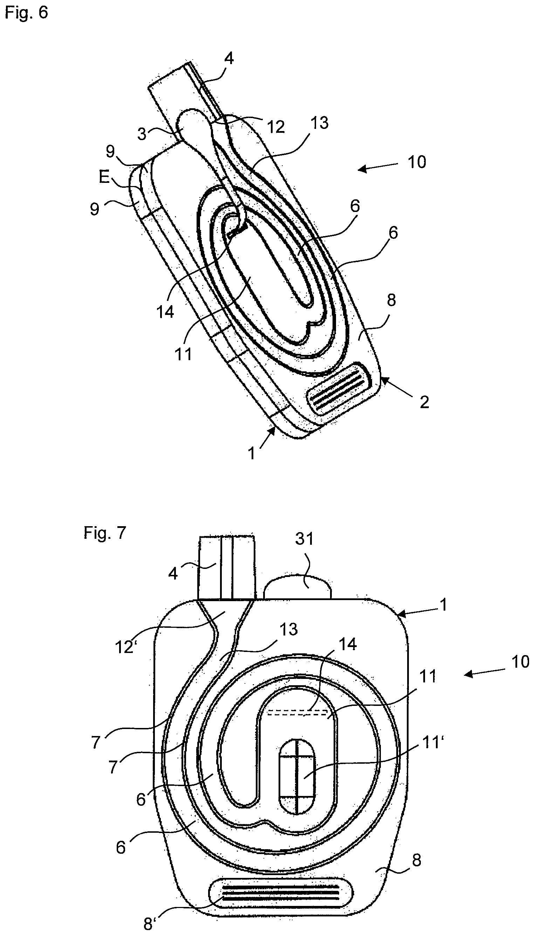

**Fig. 6 bis 9** zeigen einen erfindungsgemäßen Einzeldosis-Pulverinhalator 10, bei dem die Lufteinlassöffnung 14 nicht randständig ist, sondern in der Schalenbasis 8 des Gehäuseteils 2 schlitzförmig ausgebildet ist. Die Lufteinlassöffnung 14 mündet dabei direkt in die Medikamentenkammer 11, oberhalb der Pulveraufnahmemulde 11', die in dem anderen Gehäuseteil 1 ausgeformt ist. Auch hier erstreckt sich das Folienelement 3, das mit einer (hier nicht dargestellten Verschlusslasche) die Pulveraufnahmemulde 11' verschließt, mit der Abziehlasche 31 durch die Lufteinlassöffnung 14 aus dem Inhalator 10 heraus. Da in dieser Ausführungsform quasi kein Einlasskanal vorliegt, weist dieser Inhalator 10 keine gesonderte Pulverrückhaltestruktur auf. Auf diese kann hier durch die schlitzförmige Gestaltung der Lufteinlassöffnung 14 und deren Anordnung oberhalb der Pulveraufnahmemulde 11' verzichtet werden, da ungewollter Pulveraustritt hier auch durch den vereinfachten Abzugsvorgang des Folienelements 3 vermieden wird, bei dem ein geringerer Kraftaufwand zum Lösen und herausziehen des Folienelements 3 durch die Abzugsrichtung infolge der Anordnung der Pulveraufnahmemulde 11' gegenüber der Lufteinlassöffnung 14 und die damit verbundene kurze Abmessung des aus dem Inhalator herauszuziehenden Bandabschnitts erforderlich ist.

Es ist anzumerken, dass die gezeigte schlitzförmige Lufteinlassöffnung auch anders ausgeformt sein kann; statt eines Schlitzes kann eine andere Geometrie gewählt werden, oder es liegen weitere Öffnungen nach Art einer Bypassöffnung vor.

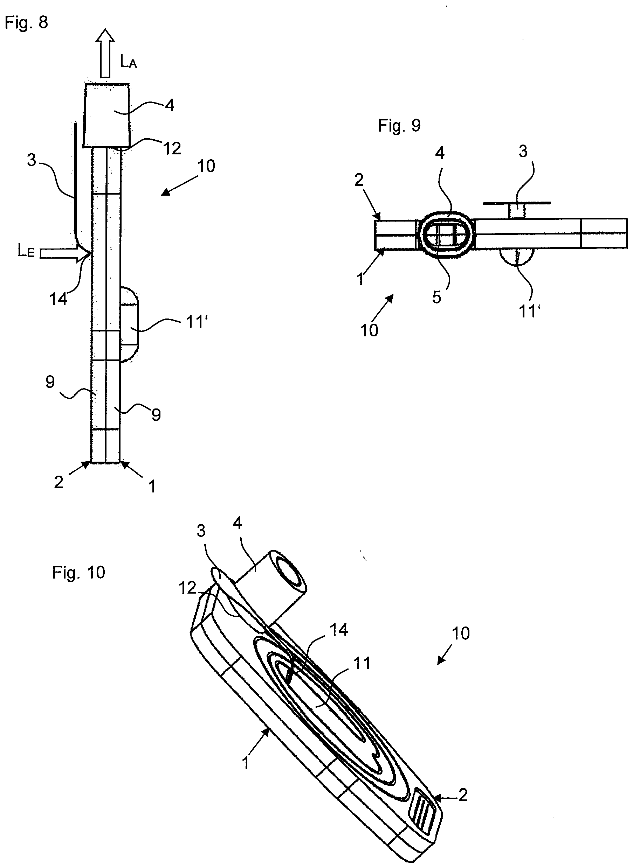

Wie die Blockpfeile in **Fig. 8** zeigen, sind hier die Lufteinlassöffnung 14 und die Auslassöffnung 12 rechtwinklig zueinander angeordnet sind, sodass die Lufteinlassströmungsrichtung L_{E} und die Luftauslassströmungsrichtung L_{A} in einem Winkel von ca. 90° zueinander verlaufen. Die in der Schalenbasis 8 liegende Lufteinlassöffnung 14 liegt dabei in einer Ebene parallel zur Verbindungsebene E, während die randständige Auslassöffnung 12 senkrecht dazu angeordnet ist.

Der Auslasskanalabschnitt 13 des Pulverinhalators aus **Fig. 6 bis 9** verläuft spiralförmig mit zwei Windungen 6 um die Medikamentenkammer 11, und bildet vor der Auslassöffnung 12 an dem Nasenstück 4 eine Aufweitung 12'.

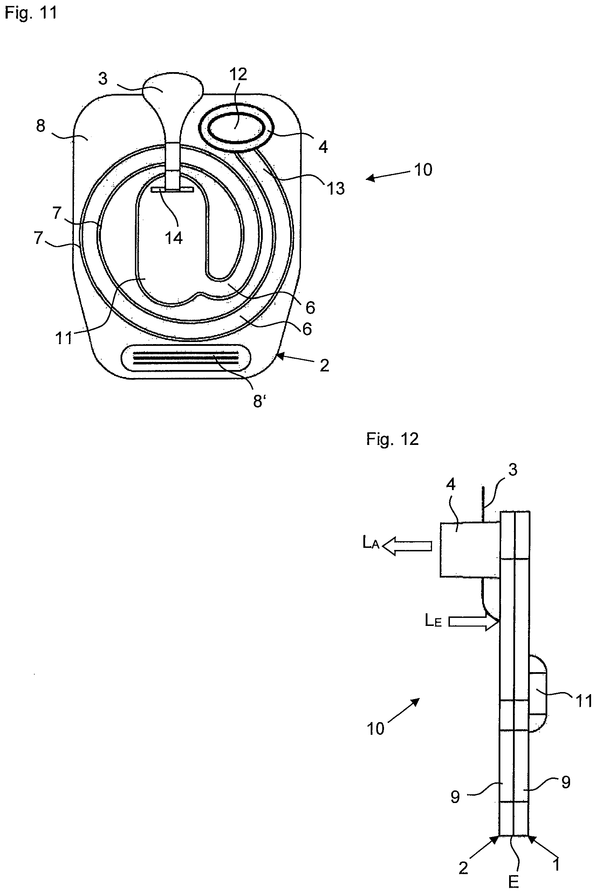

In **Fig. 10 bis 12** ist ein erfindungsgemäßer Einzeldosis-Pulverinhalator 10 gezeigt, der sich von dem Pulverinhalator aus **Fig. 6 bis 9** lediglich hinsichtlich der Anordnung der Auslassöffnung 12 und des Nasenstücks 4 unterscheidet. Die Auslassöffnung 12 liegt hier ebenfalls in der Schalenbasis 8 des Gehäuseteils 2 vor, so dass sich das an die Auslassöffnung 12 anschließende Nasenstück 4 sowie die Luftauslassrichtung L_{A} ebenso wie die entgegengesetzt verlaufende Lufteinlassrichtung L_{E} senkrecht zur Verbindungsebene E erstrecken.

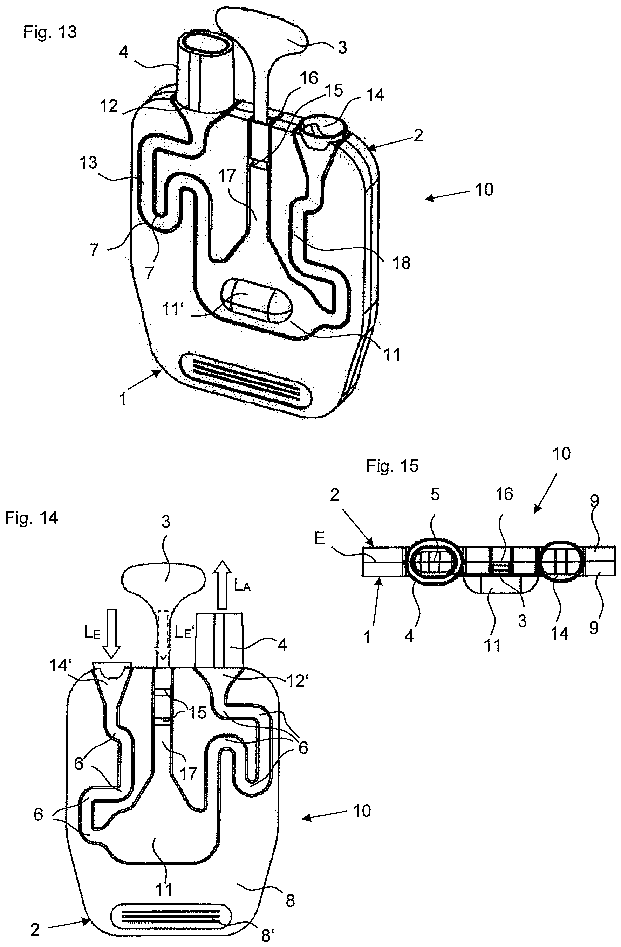

Die im Zusammenhang mit **Fig. 13 bis 15** gezeigte Ausführungsform eines erfindungsgemäßen Einzeldosis-Pulverinhalators 10 weist wiederum einen mäandrierenden Auslasskanalabschnitt 13 und auf einer Seite randständig angeordnete Lufteinlassöffnung 14 und Auslassöffnung 12 auf, sodass Luftauslassrichtung L_{A} entgegengesetzt zu Lufteinlassrichtung L_{E} verläuft. Hier weist allerdings auch der Einlasskanalabschnitt 18 mehrere Windungen 6 auf. Aufgrund der mehreren Windungen 6 kann im Einlasskanalabschnitt 18 dieser Ausführungsform auf eine weitere Pulverrückhaltestruktur verzichtet werden, allerdings verhindert der mäandrierende Einlasskanalabschnitt 18 auch das Durchziehen eines Folienelements, das zum Verschließen der Pulveraufnahmemulde 11' in der Medikamentenkammer 11 vorgesehen ist. Daher weist dieser Pulverinhalator 10 einen zusätzlichen Kanal 17 auf, der sich von einer ebenfalls randständigen Öffnung 16 zwischen der Lufteinlassöffnung 14 und der Austrittsöffnung 12 geradlinig in die Medikamentenkammer 11 erstreckt. Das die Pulveraufnahmemulde 11' verschließende Folienelement 3 erstreckt sich durch diesen Kanal 17 und mit der Abziehlasche aus der Öffnung 16, die nach Herausziehen des Folienelements 3 bei der Anwendung als zusätzliche Lufteintrittsöffnung wirkt, wie dies durch den gestrichelten Blockpfeil L_{E}' in **Fig. 14** angedeutet ist. Aufgrund des geradlinigen Verlaufs des Laschenkanals 17 ist dort zur Vermeidung ungewollten Pulveraustritts eine Pulverrückhaltestruktur 15 angeordnet. Diese Pulverrückhaltestruktur entspricht der Wallanordnung im Eintrittskanalabschnitt, die im Zusammenhang mit der in **Fig. 1 bis 5** gezeigten Ausführungsform beschrieben wurde.

Der Einzeldosis-Pulverinhalator 10, der in **Fig. 16 und 17** gezeigt ist, ist eine nicht erfindungsgemäße Ausführungsform, bei der ein Mundstück 19 an die Auslassöffnung 12 angeformt ist, sodass dieser Pulverinhalator 10 zur oral inhalativen Anwendung vorgesehen ist. Für eine turbulente Luftströmung in die und aus der Medikamentenkammer 11 sind Einlasskanalabschnitt 18 und Auslasskanalabschnitt 13 mäandrierend mit Kurven 6 ausgebildet. Zur besseren Verteilung der Pulverpartikel und gegebenenfalls auch zur Zerkleinerung von gebildeten Pulveragglomeraten sind hierbei im Auslasskanalabschnitt 13 Prallwände 20 vorgesehen. Die Auslassöffnung 12 ist wie die Einlassöffnung 14 randständig in den Gehäuseteilen 1, 2, jedoch an benachbarten Seiten ausgebildet, sodass Lufteinlassströmungsrichtung L_{E} und Luftauslassströmungsrichtung L_{A} einen Winkel von 90° bilden.

Weiter ist in **Fig. 17****,** die das Gehäuseteil 1 von innen zeigt, das komplette Folienelement 3 zu sehen, dessen Verschlusslasche 30 die dadurch nicht sichtbare Pulveraufnahmemulde verschließt. Das Folienelement 3 kann aus Aluminium, Kunststoff oder aus einer Verbundfolie beschaffen sein. Wie in **Fig. 17** zu sehen ist, erstreckt sich der von der Abziehlasche 31 kommende Bandabschnitt des Folienelements 3 bis zur distalen Seite der Verschlusslasche 30, sodass durch Zug an der Abziehlasche 31, die Verschlusslasche 30, die durch Kleben oder Siegeln an dem Gehäuseteil 1 befestigt ist, von der distalen Seite her abgezogen und dann komplett aus dem Inhalator 10 entfernt werden kann. Die in der nun geöffneten Pulveraufnahmemulde 11' enthaltene Pulverdosis wird dadurch freigesetzt und wird mit einer durch einen Einatemzug an der Auslassöffnung 12 erzeugten Luftströmung mitgerissen.

Bis auf das Mundstück entsprechen die in Zusammenhang mit **Fig. 16** und **17** beschriebenen Inhalatoren erfindungsgemäßen Ausführungsformen. D. h., dass die beschriebenen Details - bis auf das Mundstück - auf erfindungsgemäße Ausführungsformen übertragbar sind, wenn an Stelle des Mundstücks ein Nasenstück an die Auslassöffnung angeformt ist.

In entsprechender Weise sind die Auslassöffnung 12 und die Einlassöffnung 14 des in **Fig. 18** dargestellten Einzeldosis-Pulverinhalators 10 angeordnet, der allerdings ein Nasenstück 4 aufweist und somit in der bevorzugten Ausführungsform zur nasalen Applikation ausgebildet ist. Dieser Pulverinhalator 10 entspricht dem in **Fig. 1 bis 5** dargestellten bis auf die abweichende Anordnung von Auslassöffnung 12 und Einlassöffnung 14, und der dadurch bedingten zusätzlichen Kurve 6 im Auslasskanalabschnitt 13.

**Fig. 19** zeigt einen Einzeldosis-Pulverinhalator 10, bei dem im Vergleich zu dem in **Fig. 18** gezeigten, Lufteinlassöffnung 14 und Auslassöffnung 12 vertauscht sind. Anders als in den übrigen Ausführungsformen stellt hier die Pulverrückhaltestruktur 15, die hier im Auslasskanalabschnitt 13 vorliegt, mit den alternierend sich in den Kanal erstreckenden Wällen 15' die Windungen 6 (vgl. **Fig. 20)** bereit, die für die geforderten mehreren Richtungsänderungen der durch den Auslasskanalabschnitt 13 geführten Luftströmung sorgen.

Zwar sind in den mit den Figuren beispielhaft beschriebenen Ausführungsformen die Lufteinlassöffnung 14 und die Auslassöffnung 12 jeweils derart in Bezug zueinander angeordnet, dass die Lufteinlassströmungsrichtung L_{E} und die Luftauslassströmungsrichtung L_{A} in einem Winkel von 90° zueinander oder 180° (entgegengesetzt) verlaufen, es versteht sich aber von selbst, dass auch hiervon abweichende Anordnungen vorgesehen sein können, die innerhalb des Schutzumfangs der Erfindung liegen, solange Lufteinlassöffnung und Auslassöffnung nicht in der Geradeausrichtung liegen, sondern darüber hinaus vorzugsweise so angeordnet sind, dass die Lufteinlassströmungsrichtung L_{E} und die Luftauslassströmungsrichtung L_{A} einen Winkel von zumindest 90° aufspannen, um wirkungsvoll ein Herausfallen des Pulvers in der Anwendungsposition des Inhalators mit der im Wesentlichen senkrecht nach oben gerichteten Auslassöffnung zu verhindern.

Insbesondere, wenn das Inhalatorgehäuse nicht wie in den dargestellten Beispielen eine im Wesentlichen rechteckige Basis (bzw. eine aus abgerundeten Rechteck und Trapez zusammengesetzte Form), sondern beispielsweise eine ovale oder regelmäßig sechseckige Form hat, können Lufteinlassöffnung und Auslassöffnung von den dargestellten Anordnungen mit um 90° oder 180° versetzten Strömungsrichtungen abweichen.

Weiter zeigen die in den Figuren gezeigten Ausführungsbeispiele Gehäuseteile mit einer Schalenbasis 8, wobei eine Außenoberfläche des Gehäuses bis auf die Pulveraufnahmemulde und die Aufweitungen zu Einlass- und Auslassöffnung im Wesentlichen eben ist. Nicht gezeigt sind Ausführungsformen eines erfindungsgemäßen Einzeldosis-Pulverinhalators, dessen Gehäuseteile auf einer Plattenbasis beruhen. Wie sich der Fachmann aber ohne weiteres vorstellen kann, sind dann die entsprechenden Strukturen in den Gehäuseteilen anstelle in Schalenelementen in Plattenelementen ausgeformt, sodass sich die Strukturen aus der ebenen Plattenbasis erheben. Während die Gehäuseteile 1, 2 auf Schalenbasis 8 an ihren Rändern 9 und der den Luftkanal und die Medikamentenkammer 11 begrenzenden Wandung 7 miteinander verbunden werden, können Gehäuseteile auf Plattenbasis flächig in der Verbindungsebene außerhalb der ausgeformten Bereiche miteinander verbunden werden.

Die Gehäuseteile eines erfindungsgemäßen Einzeldosis-Pulverinhalators können durch Wärmeeinwirkung miteinander verschweißt werden, es kommen aber auch Verkleben, Klemmverbindungen (wobei der Rand eines Gehäuseteils um den Rand des anderen Gehäuseteils herumgebogen wird), Rastverbindungen oder Heftverbindungen in Frage.

Der erfindungsgemäße Pulverinhalator ist zur einmaligen Verwendung vorgesehen, so dass das Inhalatorgehäuse nach dem Öffnen der Pulveraufnahmemulde und der Inhalation der Pulverdosis nicht wieder verwendet werden kann, was auch dem Schutz vor Fehlanwendungen dient. Neben der guten Recyclebarkeit oder der Bioabbaubarkeit relativiert sich das aus Sicht des Umweltschutzes als nachteilig zu betrachtende erhöhte Verpackungsmüllvolumen aber auch durch den verringerten Einsatz von Multidose-Inhalatoren, deren Entsorgung aufwändiger ist, da meist Restmengen des pulverförmigen Medikaments enthalten sind.

Der erfindungsgemäße Einzeldosis-Pulverinhalator ermöglicht die wirtschaftliche Anwendung für diverse Indikationen - etwa, wenn nur wenige Anwendungen oder nur bei Bedarf erforderlich sind. Der erfindungsgemäße Pulverinhalator ist hygienisch und unerwünschte Einflüsse wie hohe Luftfeuchtigkeit oder Verschmutzung durch falsche Benutzung sind weitestgehend ausgeschlossen. Wegen der kostengünstigen Herstellung eignet sich der erfindungsgemäße Pulverinhalator auch zum Einsatz in der Dritten Welt und in Krisengebieten.

Tatsächlich ist der erfindungsgemäße Pulverinhalator so günstig in der Herstellung, dass sich eine patientenindividuelle Arzneimittelverblisterung in ökonomisch vertretbarem Rahmen realisieren lässt. Die Zusammensetzung und Dosierung des pulverförmigen Medikaments (bzw. mehrere Medikamente) lassen sich somit für jeden Patienten individuell und maßgeschneidert in dem erfindungsgemäßen Pulverinhalator abfüllen.

Der Kunststoff, der zur Herstellung eines erfindungsgemäßen Pulverinhalators, der als Wegwerfartikel konzipiert ist, verwendet wird, kann vorzugsweise ein biologisch abbaubarer Kunststoff sein. Ferner kann der Kunststoff entweder ein Kunststoff mit antiseptischen bzw. antibakteriellen und/oder antimikrobiellen Eigenschaften sein oder zumindest teilweise mit einer antiseptischen bzw. antibakteriellen und/oder antimikrobiellen Beschichtung versehen sein. So können besonders Lufteinlasskanal, Medikamentenkammer und Luftauslasskanal beschichtet sein, um in der Anwendung der Inhalationsvorrichtung keine Keime zu inhalieren. Wegen der einfacheren Applikation kann aber auch die gesamte Inhalationsvorrichtung beschichtet sein. Ein Beispiel für eine solche Beschichtung ist Perlazid ® von Rilit-Lackfabrik GmbH, Endingen, Deutschland.

Ein erfindungsgemäßer Pulverinhalator kann beispielsweise auch in einer Umverpackung vertrieben werden, die sicherstellt, dass der Pulverinhalator sauber bzw. steril und damit sofort anwendungsbereit ist. Alternativ ist eventuell denkbar, dass an Lufteinlass- und Auslassöffnung ein Deckelelement vorgesehen sein kann, mit der die Öffnungen beispielsweise nach Art eines Joghurtbecherdeckels verschlossen werden können.

Schließlich kann der zur Herstellung eines erfindungsgemäßen Pulverinhalators eingesetzte Kunststoff einen Marker zu seiner Erkennbarkeit aufweisen, um Plagiate, die den Marker nicht enthalten, erkennen zu können.

### BEZUGSZEICHENLISTE

- 1: Gehäuseteil mit Pulverkapsel
- 2: Gehäuseteil
- 3: Folienelement
- 30: Verschlusslasche
- 31: Abziehlasche
- 4: Nasenstück
- 6: Windung
- 7: Luftkanalwandung
- 8: Schalenbasis
- 8': Riffelung
- 9: Rand
- 10: Einzeldosis-Pulverinhalator
- 11: Medikamentenkammer
- 11': Pulveraufnahmemulde
- 12: Auslassöffnung
- 12': Aufweitung
- 13: Auslasskanal
- 14: Lufteinlassöffnung
- 14': Aufweitung
- 15: Pulverrückhaltestruktur
- 15': Wall
- 16: Laschenöffnung
- 17: Laschenkanal
- 18: Einlasskanal
- 19: Mundstück
- 20: Prallwand
- E: Verbindungsebene
- L_{A}: Luftauslassströmungsrichtung
- L_{E}: Lufteinlassströmungsrichtung

## Patentansprüche

1. Einzeldosis-Pulverinhalator (10) zur nasalen Applikation, der ein flaches Inhalatorgehäuse aufweist, das aus zwei flächigen, entlang einer Verbindungsebene (E) miteinander verbundenen Gehäuseteilen (1,2) besteht, in denen eine Medikamentenkammer (11) mit zumindest einer Pulveraufnahmemulde (11'), eine Lufteinlassöffnung (14) und ein durch eine Wandung (7) begrenzter Luftkanal, der sich von der Lufteinlassöffnung (14) über die Medikamentenkammer (11) zu einer Auslassöffnung (12) erstreckt, ausgeformt sind,
wobei die Lufteinlassöffnung (14) nicht auf einer von der Auslassöffnung (12) abgewandten Seite vorliegt, und der Luftkanal eine Richtungsänderung einer Luftströmung durch die Lufteinlassöffnung (14) in Bezug zu der Auslassöffnung (12) bereitstellt, und wobei an die Auslassöffnung (12) ein Nasenstück (4) angeformt ist, das zur Aufnahme in ein Nasenloch oder in beide Nasenlöcher ausgebildet ist,
**dadurch gekennzeichnet, dass**
ein Auslassabschnitt (13) des Luftkanals zwischen der Medikamentenkammer (11) und der Auslassöffnung (12) mehr als eine Windung (6) und keine Desagglomerationsstruktur aufweist.

2. Einzeldosis-Pulverinhalator (10) nach Anspruch 1,
**dadurch gekennzeichnet, dass**
die Lufteinlassöffnung (14) und die Auslassöffnung (12) nebeneinander oder um 90° oder einen dazwischen liegenden Winkel versetzt zueinander angeordnet sind.

3. Einzeldosis-Pulverinhalator (10) nach Anspruch 1 oder 2,
**dadurch gekennzeichnet, dass**
die Lufteinlassöffnung (14) und die Auslassöffnung (12) nebeneinander senkrecht oder parallel zu der Verbindungsebene (E) der Gehäuseteile (1,2) angeordnet sind, oder
dass eine von Lufteinlassöffnung (14) und Auslassöffnung (12) parallel zu der Verbindungsebene (E) der Gehäuseteile (1,2) und die entsprechend andere senkrecht zu der Verbindungsebene (E) der Gehäuseteile (1,2) angeordnet ist.

4. Einzeldosis-Pulverinhalator (10) nach zumindest einem der Ansprüche 1 bis 3,
**dadurch gekennzeichnet, dass**
der Auslassabschnitt (13) des Luftkanals zwischen der Medikamentenkammer (11) und der Auslassöffnung (12) als
- mäandrierender Kanal mit zumindest drei Kurven als Windungen (6) ausgebildet ist, oder als
- spiralförmiger Kanal mit zumindest zwei Windungen (6) ausgebildet ist.

5. Einzeldosis-Pulverinhalator (10) nach zumindest einem der Ansprüche 1 bis 4,
**dadurch gekennzeichnet, dass**
der Einzeldosis-Pulverinhalator (10) zumindest ein abziehbares Folienelement (3) aufweist, das eine Verschlusslasche (30), die die zumindest eine Pulveraufnahmemulde (11') in der Medikamentenkammer (11) verschließt, und eine Abziehlasche (31) aufweist, die sich von der Verschlusslasche (30) aus der Medikamentenkammer (11)
- durch einen Einlassabschnitt (18) des Luftkanals zwischen der Lufteinlassöffnung (14) und der Medikamentenkammer (11) aus der Lufteinlassöffnung (14) oder
- durch einen zusätzlichen Laschenkanal (17), der sich zwischen einer zusätzlichen Laschenöffnung (16) und der Medikamentenkammer (11) erstreckt, aus der Laschenöffnung (16)
heraus erstreckt.

6. Einzeldosis-Pulverinhalator (10) nach Anspruch 5,
**dadurch gekennzeichnet, dass**
der Einlassabschnitt (18) des Luftkanals und/oder der Laschenkanal (17) eine Pulverrückhaltestruktur (15) aufweist, die den Kanalquerschnitt verengt und vorzugsweise durch zumindest einen Wall (15') gebildet wird, der sich quer zur Strömungsrichtung in den Luftkanal erstreckt,
und/oder
der Einlassabschnitt (18) des Luftkanals mehr als eine Windung (6) aufweist, sodass eine durch den Einlasskanalabschnitt (18) führbare Luftströmung mehrere Richtungsänderungen vollführt.

7. Einzeldosis-Pulverinhalator (10) nach Anspruch 5 oder 6,
**dadurch gekennzeichnet, dass**
in den Gehäuseteilen (1,2) zwei oder mehr Pulveraufnahmemulden (11') für jeweils eine pulverförmige Medikamententeildosis in der Medikamentenkammer (11) ausgebildet sind, wobei die zwei oder mehr Pulveraufnahmemulden (11') durch zwei oder mehr Folienelemente (3) oder durch ein gemeinsames Folienelement (3) verschlossen sind.

8. Einzeldosis-Pulverinhalator (10) nach zumindest einem der Ansprüche 1 bis 7,
**dadurch gekennzeichnet, dass**
die Gehäuseteile (1,2)
- aus Plattenelementen gebildet sind, die flächig in der Verbindungsebene (E) außerhalb der ausgeformten Bereiche miteinander verbunden sind,
oder vorzugsweise
- aus Schalenelementen mit umlaufenden Rändern (9) gebildet sind, die in der Verbindungsebene (E) zumindest an den Rändern (9) miteinander verbunden sind.

9. Einzeldosis-Pulverinhalator nach zumindest einem der Ansprüche 1 bis 8,
**dadurch gekennzeichnet, dass**
die Gehäuseteile (1,2) jeweils Kunststoffspritzgussteile oder jeweils aus einer pharmagerechten, bevorzugt sortenreinen Kunststofffolie geformt, bevorzugt tiefgezogen sind, wobei der Kunststoff
- zumindest einer der Gehäuseteile (1,2) ein transparenter Kunststoff ist, und/oder
- biologisch abbaubar ist, und/oder
- einen Marker enthält und/oder
- ein antiseptischer und/oder antimikrobieller Kunststoff ist oder zumindest entlang dem Luftkanal eine antiseptischen und/oder antimikrobiellen Beschichtung aufweist.

## Claims

1. A single-dose powder inhaler (10) for nasal application, having a flat inhaler housing consisting of two planar housing parts (1, 2) which are connected to each other along a connecting plane (E) and in which there are formed a medicament chamber (11) with at least one powder-receiving hollow (11'), an air inlet opening (14), and an air channel delimited by a wall (7) and extending from the air inlet opening (14) via the medicament chamber (11) to an outlet opening (12),
wherein the air inlet opening (14) is not present on a side facing away from the outlet opening (12), and the air channel provides a change of direction of an air flow through the air inlet opening (14) relative to the outlet opening (12), and wherein a nosepiece (4), designed to be received in one nostril or in both nostrils, is integrally formed at the outlet opening (12),
**characterized in that**
an outlet portion (13) of the air channel between the medicament chamber (11) and the outlet opening (12) has more than one winding (6) and no deagglomerating structure.

2. The single-dose powder inhaler (10) as claimed in claim 1,
**characterized in that**
the air inlet opening (14) and the outlet opening (12) are arranged next to each other or are offset from each other by 90° or an angle lying therebetween.

3. The single-dose powder inhaler (10) as claimed in claim 1 or 2,
**characterized in that**
the air inlet opening (14) and the outlet opening (12) are arranged next to each other perpendicularly or parallel to the connecting plane (E) of the housing parts (1, 2),
or
**in that** one of air inlet opening (14) and outlet opening (12) is arranged parallel to the connecting plane (E) of the housing parts (1, 2), and the respective other is arranged perpendicularly to the connecting plane (E) of the housing parts (1, 2).

4. The single-dose powder inhaler (10) as claimed in at least one of claims 1 to 3,
**characterized in that**
the outlet portion (13) of the air channel between the medicament chamber (11) and the outlet opening (12) is designed as
- a meandering channel with at least three curves as windings (6), or as
- a spiral-shaped channel with at least two windings (6).

5. The single-dose powder inhaler (10) as claimed in at least one of claims 1 to 4,
**characterized in that**
the single-dose powder inhaler (10) has at least one tear-off film element (3) having a closure tab (30) which closes the at least one powder-receiving hollow (11') in the medicament chamber (11), and a tear-off tab (31) which, from the closure tab (30), extends out of the medicament chamber (11)
- through an inlet portion (18) of the air channel between the air inlet opening (14) and the medicament chamber (11) out of the air inlet opening (14) or
- through an additional tab channel (17) which extends between an additional tab opening (16) and the medicament chamber (11) out of the tab opening (16).

6. The single-dose powder inhaler (10) as claimed in claim 5,
**characterized in that**
the inlet portion (18) of the air channel and/or the tab channel (17) has a powder-retaining structure (15) which narrows the channel cross section and which is preferably formed by at least one barrier (15') that extends into the air channel transversely with respect to the direction of flow, and/or
the inlet portion (18) of the air channel has more than one winding (6), such that an air flow that can be guided through the inlet channel portion (18) performs several changes of direction.

7. The single-dose powder inhaler (10) as claimed in claim 5 or 6,
**characterized in that**
two or more powder-receiving hollows (11'), each for a partial dose of pulverulent medicament, are formed in the medicament chamber (11) in the housing parts (1, 2), wherein the two or more powder-receiving hollows (11') are closed by two or more film elements (3) or by one common film element (3).

8. The single-dose powder inhaler (10) as claimed in at least one of claims 1 to 7,
**characterized in that**
the housing parts (1, 2) are formed
- from plate elements which are connected to each other in a planar manner in the connecting plane (E) outside the shaped regions, or preferably
- from shell elements with circumferential edges (9), which are connected to each other in the connecting plane (E) at least at the edges (9).

9. The single-dose powder inhaler as claimed in at least one of claims 1 to 8,
**characterized in that**
the housing parts (1, 2) are each injection-molded plastic parts or are each shaped, preferably thermoformed, from a pharmaceutically acceptable, preferably single-type plastic film, wherein the plastic
- of at least one of the housing parts (1, 2) is a transparent plastic, and/or
- is biodegradable, and/or
- contains a marker, and/or
- is an antiseptic and/or antimicrobial plastic, or has an antiseptic and/or antimicrobial coating at least along the air channel.

## Revendications

1. Inhalateur de poudre à dose unique (10), destiné à l'application nasale, lequel présente un boîtier d'inhalateur plat qui se compose de deux parties de boîtier (1, 2) à surface plane, reliées entre elles le long d'un plan de jonction (E) et dans lesquelles sont moulés un réservoir de médicament (11) avec au moins une cavité de réception de poudre (11'), un orifice d'admission d'air (14) et un conduit d'air limité par une paroi (7), lequel s'étend de l'orifice d'admission d'air (14) à un orifice de sortie (12) en passant par le réservoir de médicament (11),
l'orifice d'admission d'air (14) ne se trouvant pas sur un côté opposé à l'orifice de sortie (12), et le conduit d'air assurant un changement de direction d'un flux d'air par l'orifice d'admission d'air (14) par rapport à l'orifice de sortie (12), et une pièce nasale (4) étant formée sur l'orifice de sortie (12), laquelle est conçue pour être placée dans une narine ou dans les deux narines,
**caractérisé en ce que**
un élément de sortie (13) du conduit d'air entre le réservoir de médicament (11) et l'orifice de sortie (12) présente plus qu'une spire (6) et ne présente pas de structure de désagglomération.

2. Inhalateur de poudre à dose unique (10) conformément à la revendication 1,
**caractérisé en ce que**
l'orifice d'admission d'air (14) et l'orifice de sortie (12) sont disposés l'un à côté de l'autre ou décalés l'un par rapport à l'autre à 90° ou selon un angle entre les deux.

3. Inhalateur de poudre à dose unique (10) conformément à la revendication 1 ou 2,
**caractérisé en ce que**
l'orifice d'admission d'air (14) et l'orifice de sortie (12) sont disposés l'un à côté de l'autre perpendiculairement ou parallèlement au plan de jonction (E) des parties du boîtier (1, 2)
ou bien
**en ce que** l'un de l'orifice d'admission d'air (14) et l'orifice de sortie (12) est disposé parallèlement au plan de jonction (E) des parties du boîtier (1, 2) et l'autre est disposé perpendiculairement au plan de jonction (E) des parties du boîtier (1, 2).

4. Inhalateur de poudre à dose unique (10) conformément au moins à l'une des revendications 1 à 3,
**caractérisé en ce que**
l'élément de sortie (13) du conduit d'air entre le réservoir de médicament (11) et
l'orifice de sortie (12) est conçue sous forme de
- conduit qui serpente avec au moins trois courbes comme spires (6), ou comme
- conduit en spirale avec au moins deux spires (6).

5. Inhalateur de poudre à dose unique (10) conformément au moins à l'une des revendications 1 à 4,
**caractérisé en ce que**
l'inhalateur de poudre à dose unique (10) présente au moins un élément en film (3) détachable qui présente une languette de fermeture (30) qui obture au moins une cavité de réception de poudre (11') dans le réservoir de médicament (11), ainsi qu'une languette détachable (31) sortant de la languette de fermeture (30) du réservoir de médicament (11)
- par un élément d'entrée (18) du conduit d'air entre l'orifice d'admission d'air (14) et le réservoir de médicament (11) à partir de l'orifice d'admission d'air (14) ou
- par un conduit de languette (17) supplémentaire qui s'étend entre un orifice de languette (16) supplémentaire et le réservoir de médicament (11) sortant d'un orifice de languette (16).

6. Inhalateur de poudre à dose unique (10) conformément à la revendication 5,
**caractérisé en ce que**
l'élément d'entrée (18) du conduit d'air et/ou le conduit de languette (17) présente une structure de rétention de poudre (15) qui rétrécit la section du conduit et préférablement est formée au moins par une paroi (15') qui s'étend transversalement par rapport au sens du courant dans le conduit d'air
et/ou
l'élément d'entrée (18) du conduit d'air présente plus qu'une spire (6) de façon à ce qu'un débit d'air pouvant passer par l'élément du conduit d'entrée (18) réalise plusieurs changements de direction.

7. Inhalateur de poudre à dose unique (10) conformément à la revendication 5 ou 6,
**caractérisé en ce que**
deux cavités de réception de poudre (11') ou plus sont formées respectivement pour une dose partielle de médicament sous forme de poudre dans le réservoir de médicament (11) dans les parties du boîtier (1, 2), les deux cavités de réception de poudre (11') ou plus étant fermées hermétiquement par deux éléments en film (3) ou plus ou par un élément en film (3) commun.

8. Inhalateur de poudre à dose unique (10) conformément à la revendication 1 à 7,
**caractérisé en ce que**
les parties du boîtier (1, 2)
- sont constituées d'éléments formant plaques qui sont reliés entre eux sur toute la surface dans le plan de jonction (E) en dehors des zones formées ou préférablement
- sont constituées d'éléments formant coques avec des bords périphériques (9), lesquels sont reliés entre eux au moins au niveau des bords (9) dans le plan de jonction (E).

9. Inhalateur de poudre à dose unique (10) conformément au moins à l'une des revendications 1 à 8,
**caractérisé en ce que**
les parties du boîtier (1, 2) sont respectivement des pièces en plastique moulé par injection ou sont respectivement formées, préférablement embouties à partir d'un film plastique selon les normes pharmaceutiques, préférablement d'une seule sorte, la matière plastique
- au moins de l'une des parties du boîtier (1, 2) étant une matière plastique transparente et/ou
- étant biodégradable et/ou
- contenant un marqueur et/ou
- étant une matière plastique antiseptique et/ou antimicrobienne ou présentant, au moins le long du conduit d'air, un revêtement antiseptique et/ou antimicrobien.
